# EUROPEAN PATENT APPLICATION

(11) **EP 4 756 831 A1**
(43) Date of publication of application: **10.06.2026**
(21) Application number: 24218214.5
(22) Date of filing: 06.12.2024
(51) Int. Cl.: G16H 40/63

(54) **CONTROL OF AN ELECTRONIC DEVICE**

(71) Applicant: Baxter Medical Systems GmbH + Co. KG, 07318 Saalfeld (DE)
(72) Inventor: GAJEWSKI, Tyler Benjamin, 07318 Saalfeld (DE); SMITH, Brendan Taylor, 07318 Saalfeld (DE)
(74) Representative: Reddie & Grose LLP

(57) **Abstract**

An electronic device (100) for controlling operation of an apparatus, the electronic device (100) comprising: a display (104); a data storage (108), the data storage (108) storing user interface information; and a controller (106), the controller (106) configured to: detect an apparatus that is connected to the electronic device (100); obtain user interface information associated with the apparatus from the data storage (108) when the apparatus is detected by the controller (106); and display a user interface associated with the apparatus obtained on the display (104).

## Description

### Technical field

The present invention relates to an electronic device, in particular an electronic device for controlling operation of an apparatus such as a patient support apparatus.

### Background

An apparatus such a patient support apparatus may have various functions which can be controlled by a user such as a caregiver. For example, a patient support apparatus may have a number of movable portions and movement of these portions may be operated and controlled by a user such as a caregiver operating an electronic device such as a control device.

The electronic device may be connected to the patient support apparatus via a wire or wirelessly. Typically, an electronic device is specific to, and associated with, one particular patient support apparatus.

It would be desirable to provide an electronic device which can be used as a control device and has improved functionality compared to typical electronic devices.

### Description

According to a first aspect, there is provided an electronic device for controlling operation of an apparatus, the electronic device comprising: a display; a data storage, the data storage storing user interface information; and a controller, the controller configured to: detect an apparatus that is connected to the electronic device; obtain user interface information associated with the apparatus from the data storage when the apparatus is detected by the controller; and display a user interface associated with the apparatus on the display.

Advantageously, the electronic device can display, on a single device, user interfaces for controlling operation of different apparatuses. The electronic device may allow a user to control operation of a plurality of apparatuses through use of a single electronic device rather than having to use multiple a different electronic device to control each different apparatus. Allowing a user to control multiple different apparatuses using one single device may improve the user experience and may lead to time savings due to reducing time spent by the user searching for the specific electronic device that controls operation of an apparatus.

The electronic device may be particularly advantageous in medical environments where many different apparatuses are present. A user can operate the single electronic device to remotely control many different apparatuses.

The electronic device may be a control device. The control device may be a remote control device. The electronic device may be a mobile phone (e.g. a smart phone or the like), a computer such as a personal computer, or a laptop or tablet.

The electronic device may comprise a housing. The housing may be a rigid housing. The housing may contain the other components of the electronic device.

The apparatus may be a medical apparatus. The medical apparatus may be a medical patient support apparatus.

The display may comprise a touch input surface. The display may comprise a touchscreen.

The display may comprise a touch sensor. The touch sensor may detect a touch input on the touch input surface.

The display may be a display screen.

The user interface information stored on the data storage may comprise information associated with one or more user interfaces.

The user interface information stored on the data storage may comprise one or more user interfaces. The user interface information stored on the data storage may comprise a plurality of user interfaces. The user interface information stored on the data storage may comprise at least a first user interface, a second user interface and a third user interface.

The user interface information may comprise identity information associated with one or more user interfaces. The identity information may comprise a user interface identifier associated with each of the one or more user interfaces. The user interface identifier may be a user interface number.

The user interface identifier associated with each of the one or more user interfaces may be a unique user interface identifier for each of the one or more user interfaces.

The user interface identifier associated with each of the one or more user interfaces may be associated with one apparatus. The user interface identifier associated with each of the one or more user interfaces may be associated with one or more apparatuses. The user interface identifier associated with each of the one or more user interfaces may be associated with a plurality of apparatuses.

The user interface information stored on the data storage may comprise information associated with one or more user interface elements.

The information associated with the one or more user interface elements may comprise identity information associated with the one or more user interface elements. The identity information may comprise a user interface element identifier associated with each of one or more user interface elements. The user interface element identifier may be a user interface element number.

The user interface element identifier associated with each of the one or more user interface elements may be a unique user interface element identifier for each of the one or more user elements.

The user interface element identifier associated with each of the one or more user interface elements may be associated with one apparatus. The user interface element identifier associated with each of the one or more user interface elements may be associated with one or more apparatuses. The user interface element identifier associated with each of the one or more user interface elements may be associated with a plurality of apparatuses.

The user interface information stored on the data storage may comprise store information associated with one or more apparatus features. An apparatus feature may be a feature or function which is supported by the apparatus.

The information associated with the one or more apparatus features may comprise identity information associated with the one or more apparatus features. The identity information may comprise an apparatus feature identifier associated with each of one or more apparatus features. The apparatus feature identifier may be an apparatus feature number.

The apparatus feature identifier associated with each of the one or more apparatus features may be a unique apparatus feature identifier for each of the one or more apparatus features.

The apparatus feature identifier associated with each of the one or more apparatus features may be associated with one apparatus. The apparatus feature identifier associated with each of the one or more apparatus features may be associated with one or more apparatuses. The apparatus feature identifier associated with each of the one or more apparatus features may be associated with a plurality of apparatuses.

The data storage may store information associated with one or more apparatuses. The data storage may store information associated with a plurality of apparatuses. The data storage may store information associated with at least a first apparatus, a second apparatus and a third apparatus.

The user interface information stored on the data storage may comprise information associated with one or more apparatuses. The user interface information stored on the data storage may comprise information associated with a plurality of apparatuses. The user interface information stored on the data storage may comprise information associated with at least a first apparatus, a second apparatus and a third apparatus.

The information associated with the one or more apparatuses may comprise identity information for allowing the apparatus to be identified. The identity information may comprise an apparatus identifier associated with each of the one or more apparatuses. The apparatus identifier associated with each of the one or more apparatuses may be a unique identifier for each of the one or more apparatuses. The apparatus identifier may be a reference number.

The user interface information may comprise information on one or more user interface identifiers associated with one or more apparatus identifiers. The user interface information may comprise information on one or more user interface identifiers associated with an apparatus identifier. The user interface information may comprise information on one or more apparatus identifiers associated with a user interface identifier.

The user interface information may comprise information on one or more user interface element identifiers associated with one or more apparatus identifiers. The user interface information may comprise on one or more user interface element identifiers being associated with an apparatus identifier. The user interface information may comprise information on one or more apparatus identifiers associated with a user interface element identifier.

The user interface information may comprise information on one or more apparatus feature identifiers associated with one or more user interface element identifiers. The user interface information may comprise information on one or more apparatus feature identifiers associated with a user interface element identifier. The user interface information may comprise information on one or more user interface element identifiers associated with an apparatus feature identifiers.

The data storage may comprise a single data storage device. The data storage may comprise a plurality of data storage devices.

The data storage may comprise a hard disk. The data storage may comprise non-volatile semiconductor memory.

Each of the one or more user interfaces may be a graphical user interface.

Each of the one or more user interfaces may comprise one or more user interface elements. The one or more user interface elements may comprise a plurality of user interface elements.

Each of the one or more user interface elements may be any element with which a user can interact by, for example, touching or selecting the user interface element. For example, a user interface element may comprise an image, a text field, a button, a slider and a window.

Each of the one or more user interfaces may be associated with a different apparatus In other words, the user interface associated with the apparatus may be a user interface which is specific and unique to the apparatus.

Alternatively, each of the one or more user interfaces may be associated with a plurality of different apparatuses.

One or more of the user interface elements may be associated with an apparatus.

Each of the one or more user interfaces may display available controls for its associated apparatus. Controls may be, for example, settings or functions.

The controller may be configured to obtain user interface information associated with the connected apparatus to the electronic device. The controller may be configured to obtain user interface information associated with the detected apparatus.

The controller may be configured to provide a user interface associated with the apparatus by obtaining one or more user interface elements from the data storage.

The controller may be configured to connect the electronic device to an apparatus in response to user input. For example, the user may press a button to connect the electronic device to an apparatus.

For example, when an apparatus is connected to the electronic device, the user may be prompted to select the apparatus via a menu.

The controller may be configured to automatically connect the electronic device to an apparatus. For example, the controller may be configured to connect the electronic device to any apparatus which is within a predetermined range or distance of the electronic device.

The controller may be configured to wirelessly connect the electronic device to the apparatus. The controller may be configured to wirelessly connect the electronic device to the apparatus automatically, without user input. The controller may be configured to wirelessly connect the electronic device to the apparatus via a wireless pairing protocol.

The controller may be configured to connect the electronic device to the apparatus using a wired connection. The controller may be configured to routinely scan for new wired connections with apparatuses. The controller may be configured to connect the electronic device to the apparatus using a wired connection protocol.

The controller may be configured to detect that a new apparatus is connected to the electronic device in response to the wireless pairing protocol being initialised. The controller may be configured to detect that a new apparatus is connected to the electronic device in response to the wired connection protocol being initialised.

The controller may be configured to obtain identity information associated with a connected apparatus. The controller may be configured to obtain a list of features and functions supported by the connected apparatus from the apparatus.

The controller may be configured to transmit a request to the apparatus to obtain identity information associated with the apparatus.

The controller may be configured to transmit the request to the apparatus to obtain identity information associated with the apparatus at a time the apparatus is connected to the electronic device.

The controller may be configured to transmit the request to the apparatus to obtain identity information associated with the apparatus in response to user input. For example, the user may press a button to obtain identity information associated with the apparatus.

The controller may be configured to receive identity information associated with the apparatus from the apparatus.

The identity information associated with the apparatus may comprise an apparatus identifier associated with the apparatus. The identity information associated with the apparatus may comprise a unique identifier associated with the apparatus. The apparatus identifier associated with the apparatus may be a unique reference number specific to the apparatus.

The identity information associated with the apparatus may comprise one or more feature identifiers associated with the apparatus. The one or more feature identifiers associated with the apparatus may correspond to the features or functions supported by the apparatus. Each feature identifier may correspond to a feature or function which is supported by the apparatus.

The controller may be configured to, when identity information associated with the apparatus is received from the apparatus, search the user interface information stored on the data storage.

The controller may be configured to, when identity information associated with the apparatus is received from the apparatus, search the user interface information stored on the data storage for a user interface associated with the apparatus.

The controller may be configured to search the user interface information stored on the data storage for a user interface identifier based on the identity information associated with the apparatus.

The controller may be configured to obtain a user interface from the user interface information stored on the data storage corresponding to the user interface identifier. The controller may be configured to display the user interface obtained from the data storage on the display.

The controller may be configured to, when identity information associated with the apparatus is received from the apparatus, search the user interface information stored on the data storage for one or more user interface elements associated with the apparatus. The controller may be configured to, when identity information associated with the apparatus is received from the apparatus, search the user interface information stored on the data storage for one or more user interface elements associated with the identity information.

The controller may be configured to obtain one or more user interface elements associated with the apparatus from the user interface information stored on the data storage. The controller may be configured to obtain a plurality of user interface elements associated with the apparatus from the user interface information stored on the data storage.

The controller may be configured to obtain one or more user interface elements associated with the apparatus identity information from the user interface information stored on the data storage. The controller may be configured to obtain a plurality of user interface elements associated with the apparatus identity information from the user interface information stored on the data storage.

The controller may be configured to compile a user interface comprising the obtained user interface elements. The controller may be configured to display a user interface comprising the obtained user interface elements on the display. The controller may be configured to display the compiled user interface on the display.

The controller may be configured to display a user interface on the display based on the user interface information obtained from the data storage.

The electronic device may comprise a power source for supplying power to the electronic device. The power source may comprise a battery. The battery may comprise a rechargeable battery.

The electronic device may comprise a transceiver for sending and receiving information to and from the apparatus.

According to a second aspect, there is provided a system comprising an apparatus and an electronic device for controlling operation of the apparatus, the electronic device comprising: a display; a data storage, the data storage storing user interface information; and a controller, the controller configured to: detect that the apparatus is connected to the electronic device; obtain user interface information associated with the apparatus from the data storage when the apparatus is detected by the controller; and display a user interface associated with the apparatus on the display.

The electronic device of the second aspect may comprise any feature as described above with respect to the electronic device of the first aspect.

The apparatus of the second aspect may comprise any feature as described above with respect to the apparatus of the first aspect.

According to a third aspect, there is provided a method of operating an electronic device, the method comprising: detecting when an apparatus is connected to the electronic device; obtaining user interface information associated with the connected apparatus; and displaying a user interface associated with the apparatus.

The method may comprise connecting the electronic device to an apparatus in response to user input.

The method may comprise connecting the electronic device to an apparatus when the apparatus is within a predetermined range. The method may comprise automatically connecting the electronic device to an apparatus when the apparatus is within a predetermined range.

The method may comprise transmitting a request to the apparatus to obtain identity information associated with the apparatus.

The method may comprise transmitting the request to the apparatus to obtain identity information associated with the apparatus at a time the apparatus is connected to the electronic device.

The method may comprise transmitting the request to the apparatus to obtain identity information associated with the apparatus in response to user input. For example, the user may press a button to obtain identity information associated with the apparatus.

The method may comprise receiving identity information associated with the apparatus from the apparatus.

The identity information associated with the apparatus may comprise an apparatus identifier associated with the apparatus. The apparatus identifier associated with the apparatus may be a unique reference number specific to the apparatus.

The identity information associated with the apparatus may comprise one or more feature identifiers associated with the apparatus. The one or more feature identifiers associated with the apparatus may correspond to the features or functions supported by the apparatus. Each feature identifier may correspond to a feature or function which is supported by the apparatus.

The method may comprise obtaining user interface information associated with the connected apparatus from a data storage of the electronic device.

The method may comprise, when identity information associated with the apparatus is received from the apparatus, searching the user interface information stored on a data storage of the electronic device for a user interface associated with the apparatus. The method may comprise searching the user interface information stored on a data storage of the electronic device for a user interface identifier based on the identity information associated with the apparatus.

The method may comprise obtaining a user interface from the user interface information stored on a data storage of the electronic device corresponding to the user interface identifier.

The method may comprise displaying the obtained user interface on a display of the electronic device.

The method may comprise, when identity information associated with the apparatus is received from the apparatus, searching the user interface information stored on a data storage of the electronic device for one or more user interface elements associated with the apparatus.

The method may comprise, when identity information associated with the apparatus is received from the apparatus, searching the user interface information stored on a data storage of the electronic device for one or more user interface elements associated with the identity information.

The method may comprise obtaining one or more user interface elements associated with the apparatus from the user interface information stored on a data storage of the electronic device. The method may comprise obtaining a plurality of user interface elements associated with the apparatus from the user interface information stored on a data storage of the electronic device

The method may comprise obtaining one or more user interface elements associated with the apparatus identity information from the user interface information stored on a data storage of the electronic device. The method may comprise obtaining a plurality of user interface elements associated with the apparatus identity information from the user interface information stored on a data storage of the electronic device.

The method may compiling a user interface comprising the obtained user interface elements. The method may comprise displaying a user interface comprising the obtained one or more user interface elements on the display. The method may comprise displaying the compiled user interface on the display.

The method may comprise displaying a user interface on the display based on the user interface information obtained from the data storage.

The electronic device of the third aspect may comprise any feature as described above with respect to the electronic device of the first aspect.

According to a fourth aspect, there is provided a computer program comprising instructions such that when the computer program is execute on an electronic device, the electronic device is arranged to: detect when an apparatus is connected to the electronic device; obtain a user interface associated with the connected apparatus from a data storage of the electronic device; and display the obtained user interface on a display of the electronic device.

The electronic device of the fourth aspect may comprise any feature as described above with respect to the electronic device of the first aspect.

### Brief Description of the Drawings

Specific embodiments will now be described, by way of example only, with reference to the following examples and the accompanying drawings, in which:
Figure 1 shows schematically an example of an electronic device according to the invention;
Figure 2 shows schematically an example of user interface displayed on a display of the electronic device shown in Figure 1;
Figure 3 shows schematically an example of a system comprising the electronic device shown in Figure 1 and an apparatus;
Figure 4 shows schematically a first example of a table stored on the data storage of the electronic device shown in Figure 1;
Figure 5 shows schematically a first example of the controller of the electronic device shown in Figure 1 obtaining user interface information associated with the apparatus and displaying a user interface on the display;
Figure 6 shows schematically a second example of a table stored on the data storage of the electronic device shown in Figure 1;
Figure 7 shows schematically a second example of the controller of the electronic device shown in Figure 1 obtaining user interface information associated with the apparatus and displaying a user interface on the display;
Figure 8 shows schematically a third example of a table stored on the data storage of the electronic device shown in Figure 1; and
Figure 9 shows schematically a third example of the controller of the electronic device shown in Figure 1 obtaining user interface information associated with the apparatus and displaying a user interface on the display..

### Detailed Description

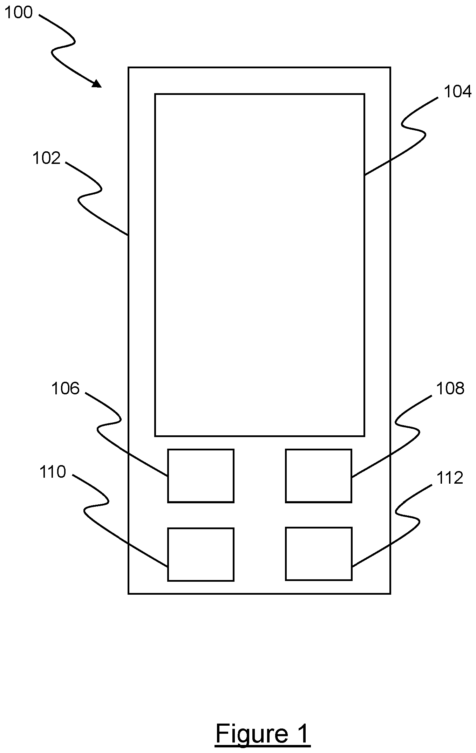

Referring to Figure 1, there is shown schematically an illustration of an electronic device 100 for controlling operation of an apparatus.

The electronic device 100 may be any type of electronic device. The apparatus may be any type of apparatus. In the example of Figure 1, the electronic device 100 is a control device, specifically a remote control device. In the example of Figure 1, the apparatus is a medical apparatus, specifically a patient support apparatus.

The electronic device 100 is configured to control operation of an apparatus in response to user input.

The electronic device 100 has a housing 102 and includes a display 104 and a controller 106.

The display 102 is a touch screen display. In other words, the display 104 comprises a touch screen which is responsive to touch input.

The electronic device 100 includes a data storage 108.

The data storage 108 stores information on one or more apparatuses. In the example of Figure 1, the data storage 108 stores information on at least a first apparatus, a second apparatus and a third apparatus.

The data storage 108 stores user interface information. In the example of Figure 1, the data storage 108 stores at least information on at least a first user interface associated with the first apparatus, a second user interface associated with the second apparatus, and a third user interface associated with the third apparatus.

In other examples, the data storage 108 may store information on additional apparatuses and additional user interfaces.

The controller 106, which may be a processor or the like, is provided within the housing 102 and is for controlling operation of the electronic device 100. The controller 106 is connected to the display 104 and the data storage 108.

The controller 106 is configured to display a user interface associated with a connected apparatus on the display 104, so as to allow a user to control operation of the apparatus by operating the displayed user interface.

The controller 106 is configured to detect an apparatus that is connected to the electronic device 100. The controller 106 is also configured to obtain user interface information associated with the apparatus from the data storage when the apparatus is detected by the controller. The controller 106 is also configured to display a user interface associated with the apparatus on the display.

The electronic device 100 also has a power source such as a battery 110 for supplying power to the electronic device 100. The battery may be a rechargeable battery.

The electronic device 100 has a transceiver 112 for sending and receiving information and communication signals to and from an apparatus.

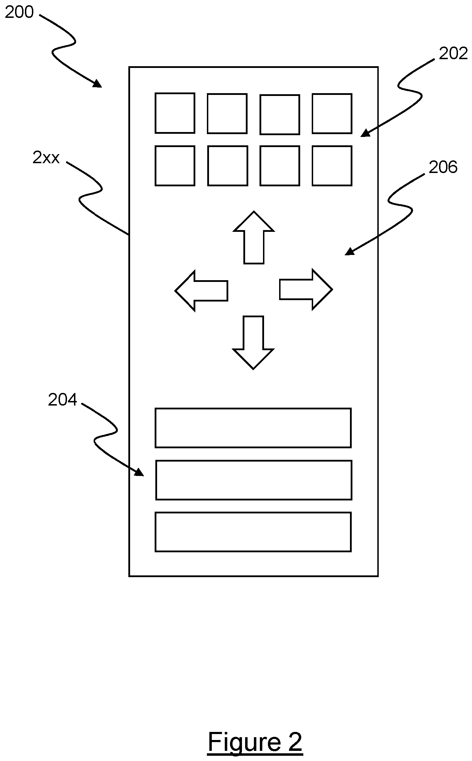

Referring to Figure 2, there is shown schematically an example of a user interface 200 which is stored in the data storage 108.

The user interface 200 in this example is a graphical user interface having a plurality of user interface elements. A user interface element is a part of the user interface which the user can interact with, for example, by touching or selecting the user interface element. Examples of user interface elements include, for example, images, text fields, buttons, sliders and windows.

In the example of Figure 2, the user interface 200 includes a plurality of buttons 202, a plurality of sliders 204 and a four arrow shaped buttons 206.

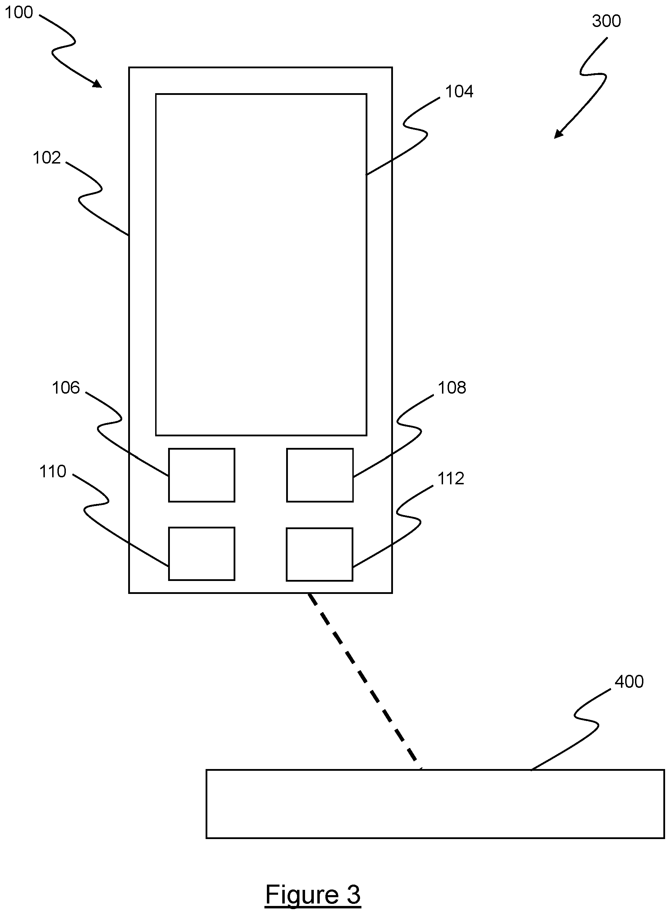

Referring to Figure 3, there is shown schematically a system 300 comprising the electronic device 100 as described with reference to Figures 1 and 2, and an apparatus 400.

In this example, the electronic device 100 is a remote control device and the apparatus 400 is a patient support apparatus.

The electronic device 100 is configured to control operation of the apparatus 400 in response to user input.

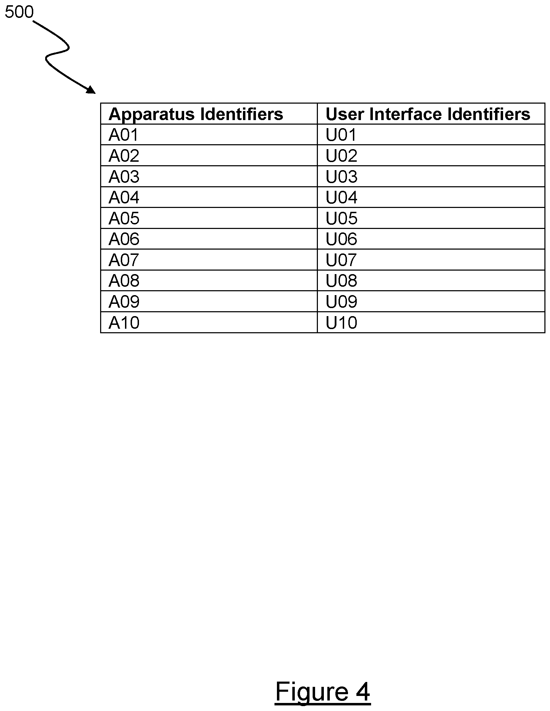

Referring to Figure 4 there is shown an example of a table 500 stored in the data storage 108. The table 500 includes user interface information. The user interface information allows the controller 106 to obtain and display a user interface associated with a connected apparatus on the display 104.

The table 500 includes identity information associated with a plurality of apparatuses. In this example, identity information comprises a plurality of "Apparatus Identifiers". The "Apparatus Identifiers" are unique identifiers associated with a plurality of apparatuses. Each apparatus is associated with a different unique one of the "Apparatus Identifiers".

The table 500 also includes identity information associated with a plurality of user interfaces. In this example, the identity information comprises a plurality of "User Interface Identifiers". The "User Interface Identifiers" are unique numbers corresponding to a plurality of user interfaces stored in the data storage 108. Each user interface is associated with a different one of the "User Interface Identifiers".

In this example, each of the "Apparatus Identifiers" has an associated one of the "User Interface Identifiers". For example, apparatus identifier "A01" is associated with user interface identifier "U01".

In other examples, each of the "User Interface Identifiers" may be associated with a plurality of different "Apparatus Identifiers".

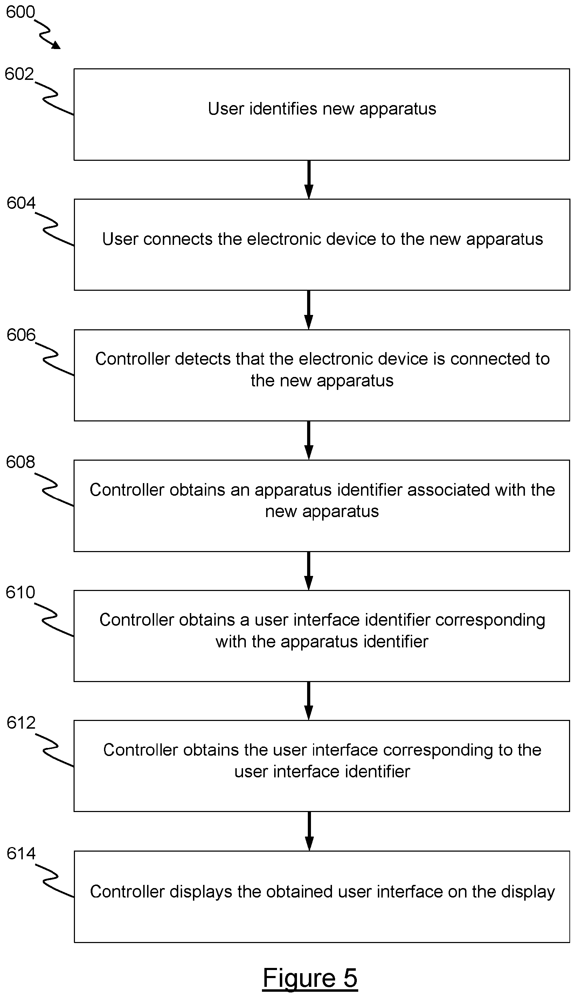

Referring to Figure 5, there is shown an example of a method 600 of the controller 106 of the electronic device 100 detecting an apparatus 400, obtaining user interface information associated with the apparatus 400, and displaying a user interface associated with the apparatus 400 on the display 104.

Initially, the electronic device 100 is not connected to an apparatus such as the apparatus 400.

At 602, a user identifies a new apparatus 400 which they would like to control the operation of.

At 604, the user connects the electronic device 100 to the new apparatus 400.

In the example of Figure 5, the user operates the electronic device 100 to scan the local area for new apparatuses within range. When the electronic device 100 finds the new apparatus 400, the user operates the electronic device 100 to connect wirelessly to the new apparatus 400 using a wireless pairing protocol. Alternatively, the electronic device 100 may automatically connect to the new apparatus 400 when it is found.

At 606, the controller 106 detects that the electronic device 100 is connected to the new apparatus 400.

In the example of Figure 5, the controller 106 detects that the new apparatus 400 is connected to the electronic device 100 due to use of the wireless pairing protocol.

At 608, the controller 106, having detected that the electronic device 100 is connected to a new apparatus 400, determines the identity information associated with the apparatus 400. The identity information may be, for example, a unique apparatus identifier associated with the apparatus 400.

In the example of Figure 5, the controller 106 determines identity information associated with the apparatus 400 by obtaining an apparatus identifier associated with the apparatus 400 from the apparatus 400. The apparatus identifier may be a unique identifier which allows the controller 106 to identify the new apparatus 400.

In this example, the controller 106 obtains the apparatus identifier "A05" from the apparatus 400.

At 610, the controller 106, having obtained an apparatus identifier associated with the apparatus 400, obtains a corresponding user interface identifier associated with the apparatus 400 from the table 300 stored in the data storage 108. In particular, the controller 106 obtains a user interface identifier corresponding to the apparatus identifier obtained from the apparatus 400. In this example, controller 106 obtains the user interface identifier "U05", which corresponds to the apparatus identifier "A05".

At 612, the controller 106 obtains the user interface corresponding to obtained user interface identifier from the data storage 108. In this example, the controller 106 obtains the user interface corresponding to user interface identifier "U05".

At 614, the controller 106 displays the obtained user interface on the display 104. In this example, the controller 106 displays the user interface corresponding to user interface identifier "U05" on the display 104.

The user is then able to control operation of the apparatus 400 by operating the user interface displayed on the display.

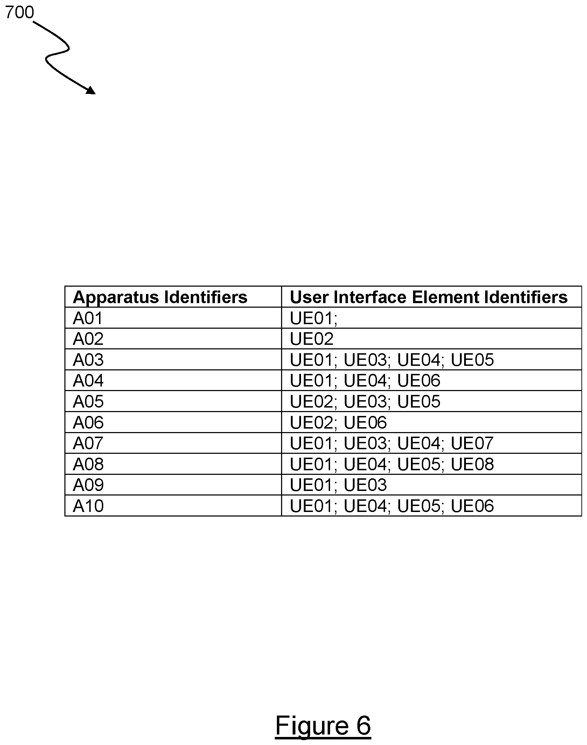

Referring to Figure 6 there is shown an example of a table 700 which may be stored in the data storage 108. The table 700 includes user interface information. The user interface information allows the controller 106 to provide and display a user interface associated with a connected apparatus on the display 104.

The table 700 includes identity information associated with a plurality of apparatuses. In this example, identity information comprises a plurality of "Apparatus Identifiers". The "Apparatus Identifiers" are unique identifiers associated with a plurality of apparatuses. Each apparatus is associated with a different unique one of the "Apparatus Identifiers".

The table 700 also includes identity information associated with a plurality of user interface elements. In this example, the identity information comprises a plurality of "User Interface Element Identifiers". The "User Interface Element Identifiers" are unique numbers corresponding to a plurality of user interface elements stored in the data storage 108. Each user interface element is associated with a different one of the "User Interface Element Identifiers".

In this example, each of the "Apparatus Identifiers" is associated with one or more of the "User Interface Element Identifiers". For example, apparatus identifier "A01" is associated with user interface element identifier "UE01".

In other examples, each of the "Apparatus Identifiers" may be associated with a plurality of different "User Interface Element Identifiers". For example, apparatus identifier "A06" is associated with user interface element identifiers "UE02" and "UE06".

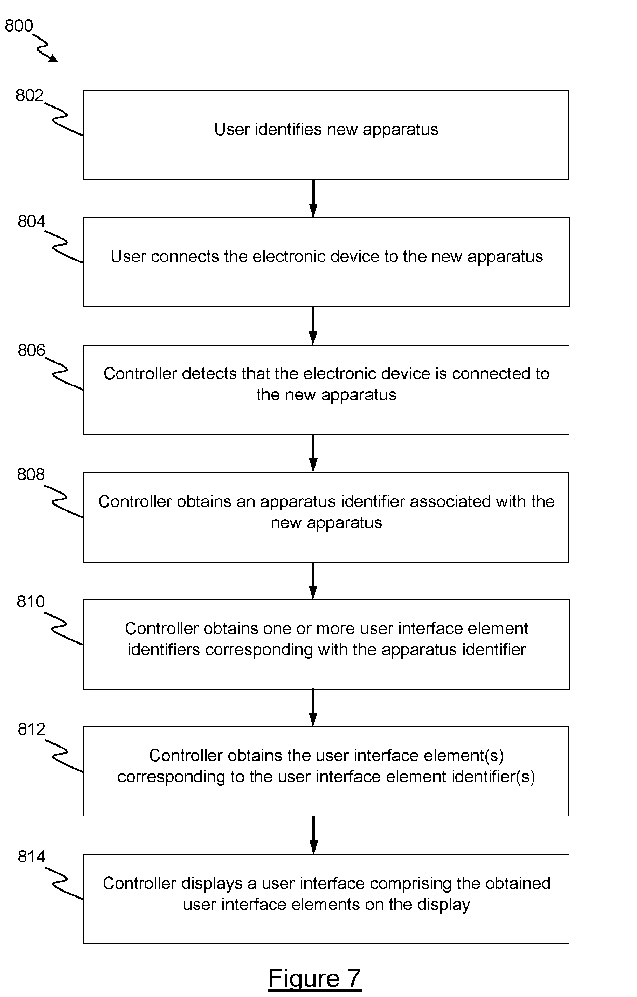

Referring to Figure 7, there is shown an example of a method 800 of the controller 106 of the electronic device 100 detecting an apparatus 400, obtaining user interface information associated with the apparatus 400, and displaying a user interface associated with the apparatus 400 on the display 104.

Initially, the electronic device 100 is not connected to an apparatus such as the apparatus 400.

At 802, a user identifies a new apparatus 400 which they would like to control the operation of.

At 804, the user connects the electronic device 100 to the new apparatus 400.

In the example of Figure 7, the user operates the electronic device 100 to scan the local area for new apparatuses within range. When the electronic device 100 finds the new apparatus 400, the user operates the electronic device 100 to connect wirelessly to the new apparatus 400 using a wireless pairing protocol. Alternatively, the electronic device 100 may automatically connect to the new apparatus 400 when it is found.

At 806, the controller 106 detects that the electronic device 100 is connected to the new apparatus 400.

In the example of Figure 7, the controller 106 detects that the new apparatus 400 is connected to the electronic device 100 due to use of the wireless pairing protocol.

At 808, the controller 106, having detected that the electronic device 100 is connected to a new apparatus 400, determines the identity information associated with the apparatus 400. The identity information may be, for example, a unique apparatus identifier associated with the apparatus 400.

In the example of Figure 7, the controller 106 determines identity information associated with the apparatus 400 by obtaining an apparatus identifier associated with the apparatus 400 from the apparatus 400. The apparatus identifier may be a unique identifier which allows the controller 106 to identify the new apparatus 400.

In this example, the controller 106 obtains the apparatus identifier "A05" from the apparatus 400.

At 810, the controller 106, having obtained an apparatus identifier associated with the apparatus 400, obtains one or more corresponding user interface element identifiers associated with the apparatus 400 from the table 700 stored in the data storage 108. In particular, the controller 106 obtains user interface element identifiers corresponding to the apparatus identifier obtained from the apparatus 400. In this example, controller 106 obtains the user interface element identifiers "UE02", "UE03" and "UE05", which correspond to the apparatus identifier "A05".

At 812, the controller 106 obtains the user interface elements corresponding to obtained user interface element identifiers from the data storage 108. In this example, the controller 106 obtains the user interface elements corresponding to user interface element identifiers "UE02", "UE03" and "UE05".

At 814, the controller 106 displays a user interface comprising the user interface elements obtained from the data storage 108 on the display 104. In this example, the controller 106 displays a user interface which comprises user interface elements corresponding to user interface element identifiers "UE02", "UE03" and "UE05" on the display 104.

The user is then able to control operation of the apparatus 400 by operating the user interface displayed on the display.

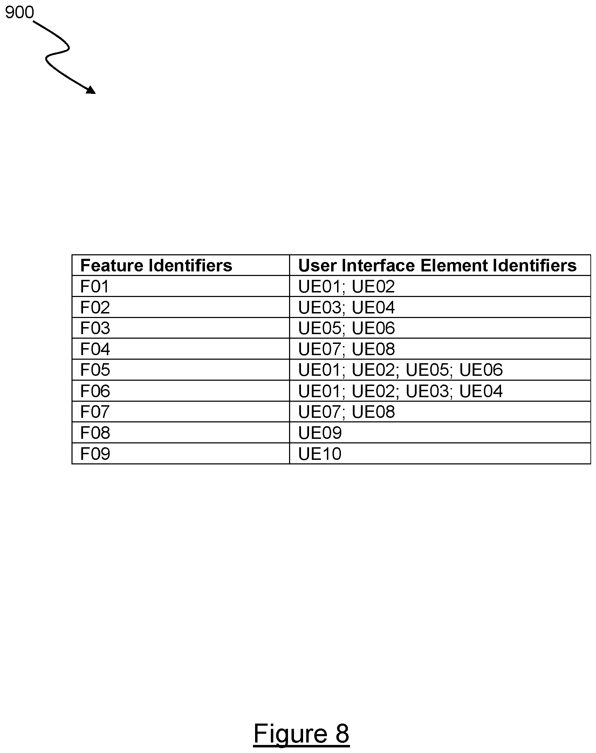

Referring to Figure 8 there is shown an example of a table 900 which may be stored in the data storage 108. The table 900 includes user interface information. The user interface information allows the controller 106 to provide and display a user interface associated with a connected apparatus on the display 104.

The table 900 includes identity information associated with one or more features which are supported by the apparatus. In this example, identity information comprises a plurality of "Feature Identifiers". The "Feature Identifiers" are unique identifiers associated with a plurality of features. Each feature is associated with a different unique one of the "Feature Identifiers".

The table 900 also includes identity information associated with a plurality of user interface elements. In this example, the identity information comprises a plurality of "User Interface Element Identifiers". The "User Interface Element Identifiers" are unique numbers corresponding to a plurality of user interface elements stored in the data storage 108. Each user interface element is associated with a different one of the "User Interface Element Identifiers".

In this example, each of the "Feature Identifiers" is associated with one or more of the "User Interface Element Identifiers". For example, feature identifier "F08" is associated with user interface element identifier "UE09".

In other examples, each of the "Feature Identifiers" may be associated with a plurality of "User Interface Element Identifiers". For example, feature identifier "F01" is associated with user interface element identifiers "UE01" and "UE02".

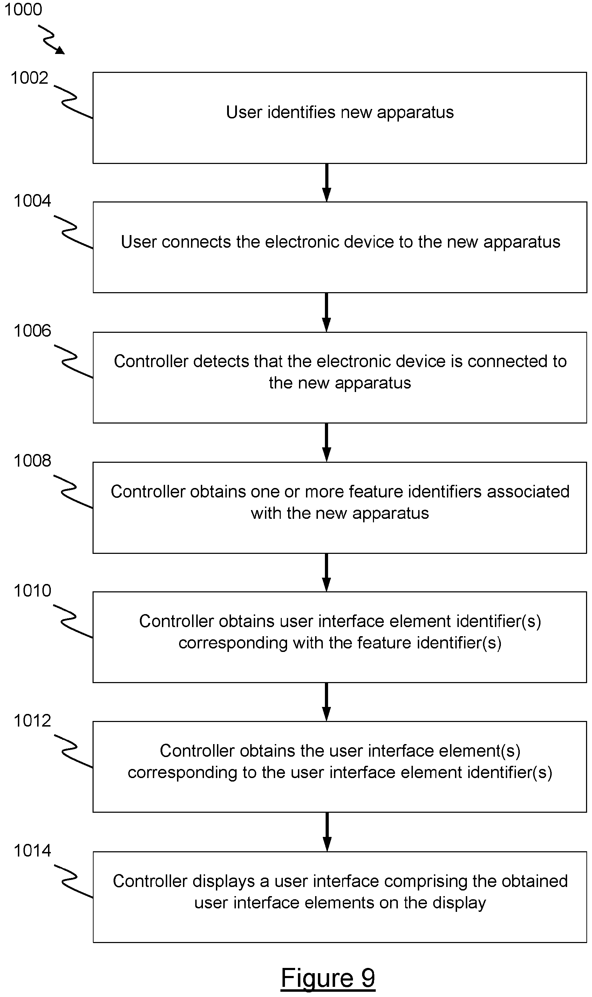

Referring to Figure 9, there is shown an example of a method 1000 of the controller 106 of the electronic device 100 detecting an apparatus 400, obtaining user interface information associated with the apparatus 400, and displaying a user interface associated with the apparatus 400 on the display 104.

Initially, the electronic device 100 is not connected to an apparatus such as the apparatus 400.

At 1002, a user identifies a new apparatus 400 which they would like to control the operation of.

At 1004, the user connects the electronic device 100 to the new apparatus 400.

In the example of Figure 9, the user operates the electronic device 100 to scan the local area for new apparatuses within range. When the electronic device 100 finds the new apparatus 400, the user operates the electronic device 100 to connect wirelessly to the new apparatus 400 using a wireless pairing protocol. Alternatively, the electronic device 100 may automatically connect to the new apparatus 400 when it is found.

At 1006, the controller 106 detects that the electronic device 100 is connected to the new apparatus 400.

In the example of Figure 9, the controller 106 detects that the new apparatus 400 is connected to the electronic device 100 due to use of the wireless pairing protocol.

At 1008, the controller 106, having detected that the electronic device 100 is connected to a new apparatus 400, determines the identity information associated with the apparatus 400. The identity information may be, for example, one or more unique identifiers associated with the apparatus 400.

In the example of Figure 9, the controller 106 determines identity information associated with the apparatus 400 by obtaining one of more feature identifiers associated with the apparatus 400 from the apparatus 400. The feature identifiers may be unique identifiers which allow the controller 106 to identify which features are supported by the new apparatus 400.

In this example, the controller 106 obtains the feature identifiers "F01" and "F04" from the apparatus 400.

At 1010, the controller 106, having obtained one or more feature identifiers associated with the apparatus 400, obtains one or more corresponding user interface element identifiers associated with the apparatus 400 from the table 900 stored in the data storage 108. In particular, the controller 106 obtains user interface element identifiers corresponding to the one or more feature identifiers obtained from the apparatus 400. In this example, controller 106 obtains the user interface element identifiers "UE01", "UE02", which correspond to the feature identifier "F01", and the user interface element identifiers "UE07" and "UE08", which correspond to the feature identifier "F04".

At 1012, the controller 106 obtains the user interface elements corresponding to obtained user interface element identifiers from the data storage 108. In this example, the controller 106 obtains the user interface elements corresponding to user interface element identifiers "UE01", "UE02", "UE07" and "UE08".

At 1014, the controller 106 displays a user interface comprising the user interface elements obtained from the data storage 108 on the display 104. In this example, the controller 106 displays a user interface which comprises user interface elements corresponding to user interface element identifiers "UE01", "UE02", "UE07" and "UE08" on the display 104.

The user is then able to control operation of the apparatus 400 by operating the user interface displayed on the display.

Advantageously, when a new apparatus is connected, the controller 106 automatically, without user input, obtains and displays a user interface which the user can operate to control the apparatus.

The electronic device 100 described above is particularly advantageous in medical environments where many apparatuses are present. A user can operate the single electronic device 100 to remotely control many different apparatuses. Use of a single electronic device eliminates the need to have a separate remote control device for each apparatus.

It will be understood that the controller 106 referred to herein may in practice be provided by a single chip or integrated circuit or plural chips or integrated circuits, optionally provided as a chipset, an application-specific integrated circuit (ASIC), field-programmable gate array (FPGA), digital signal processor (DSP), graphics processing units (GPUs), etc. The chip or chips may comprise circuitry (as well as possibly firmware) for embodying at least one or more of a data processor or processors and a digital signal processor or processors, which are configurable so as to operate in accordance with the exemplary embodiments.

Reference is made herein to data storage for storing data. This may be provided by a single device or by plural devices. Suitable devices include for example a hard disk and non-volatile semiconductor memory.

The examples described herein are to be understood as illustrative examples of embodiments of the invention. Further embodiments and examples are envisaged. Any feature described in relation to any one example or embodiment may be used alone or in combination with other features. In addition, any feature described in relation to any one example or embodiment may also be used in combination with one or more features of any other of the examples or embodiments, or any combination of any other of the examples or embodiments. Furthermore, equivalents and modifications not described herein may also be employed within the scope of the invention, which is defined in the claims.

## Claims

1. An electronic device for controlling operation of an apparatus, the electronic device comprising:
a display;
a data storage, the data storage storing user interface information; and
a controller, the controller configured to:
detect an apparatus that is connected to the electronic device;
obtain user interface information associated with the apparatus from the data storage when the apparatus is detected by the controller; and
display a user interface associated with the apparatus on the display.

2. An electronic device according to claim 1, wherein the electronic device is a control device.

3. An electronic device according to any preceding claim, wherein the apparatus is a medical apparatus.

4. An electronic device according to any preceding claim, wherein the display comprises a touchscreen.

5. An electronic device according to any preceding claim, wherein each of the one or more user interfaces is a graphical user interface.

6. An electronic device according to any preceding claim, wherein each of the one or more user interfaces comprises one or more user interface elements.

7. An electronic device according to claim 6, wherein the one or more user interface elements comprise at least one of: an image, a text field, a button, or a slider.

8. An electronic device according to any preceding claim, wherein the user interface information comprises one or more user interfaces associated with the apparatus.

9. An electronic device according to any preceding claim, wherein the user interface information comprises one or more user interface elements associated with the apparatus .

10. An electronic device according to any preceding claim, wherein the controller is configured to connect the electronic device to an apparatus in response to user input.

11. An electronic device according to any one of claims 1 to 9, wherein the controller is configured connect the electronic device to any apparatus which is within a predetermined range or distance of the electronic device.

12. An electronic device according to any preceding claim, wherein controller is configured to obtain identity information associated with a connected apparatus.

13. An electronic device according to claim 12, wherein the identity information associated with a connected apparatus comprises a unique identifier associated with the apparatus.

14. An electronic device according to any preceding claim, wherein the data storage stores identity information on one or more apparatuses and identity information on one or more user interfaces.

15. A system comprising: an electronic device according to any preceding claim; and an apparatus.
